# EUROPEAN PATENT SPECIFICATION

(11) **EP 2 255 791 B1**
(45) Date of publication and mention of the grant of the patent: **16.11.2011**
(21) Application number: 09159296.4
(22) Date of filing: 04.05.2009
(51) Int. Cl.: A61K 9/16, A61K 9/20, A61K 31/135

(54) **Extended release pharmaceutical composition comprising metoprolol succinate**
Pharmazeutische Zusammensetzung mit verlängerter Freisetzung mit Metoprololsuccinat
Composition pharmaceutique à libération prolongée comprenant du succinate de métoprolol

(30) Priority: 03.04.2009 EP 09157343
(43) Date of publication of application: 01.12.2010
(73) Proprietor: Zaklady Farmaceutyczne Polpharma SA, 83-200 Starogard Gdanski (PL)
(72) Inventor: Shah, Chitra, 400 076, Mumbai (IN); Gual-Pujol, Francisco, 08902, L'Hospitalet de Llobregat (ES); Amela-Navarro, Joaquim, 08902, L'Hospitalet de Llobregat (ES); Ruiz-Córdoba, José Luis, 08902, L'Hospitalet de Llobregat (ES)
(74) Representative: Pons Ariño, Angel

(56) References cited:
- EP-A- 0 220 143
- EP-A- 0 311 582
- WO-A-2004/069234
- WO-A-2007/141593
- WO-A-2008/012346
- US-A1- 2005 008 701
- FONNER D E ET AL: "Granulation and Tablet characteristics" PHARMACEUTICAL DOSAGE FORMS-TABLETS, XX, XX, vol. 2, 1 January 1981 (1981-01-01), pages 185-205,260, XP002993505
- RAVISHANKAR ET AL: "Modulated release metoprolol succinate formulation based on ionic interactions: In vivo proof of concept" JOURNAL OF CONTROLLED RELEASE, ELSEVIER, AMSTERDAM, NL, vol. 111, no. 1-2, 10 March 2006 (2006-03-10), pages 65-72, XP005308245 ISSN: 0168-3659

## Description

### FIELD OF THE INVENTION

The present invention relates to extended release pharmaceutical compositions comprising metoprolol succinate having a small particle size.

### BACKGROUND OF THE INVENTION

Metoprolol is a selective β1 receptor blocker used in treatment of several diseases of the cardiovascular system, especially hypertension. Metoprolol is marketed in the form of succinate salt.

European patent application EP 293347-A describes, for the first time, metoprolol succinate and an oral pharmaceutical composition which comprises a core containing a therapeutically active compound coated with a layer comprising a) 10 to 85% by weight of an anionic polymer soluble at a pH above 5.5, and b) 15 to 90% by weight of a water-insoluble polymer selected from quaternary ammonium-substituted acrylic polymers.

WO2008012346 A discloses metoprolol succinate coated granules coated with a film-former coating agent consisting of granules having a particle size ranging from 0.2 to 2 mm, friability lower than or equal to 1 % and comprising metoprolol succinate as active ingredient in an amount ranging from 10 to 75% by weight of the granule and at least one binder selected from microcrystalline cellulose and methylcellulose. These granules are used to prepare extended-release pharmaceutical compositions. Particle size of the granules ranging from 0.2 to 1 mm are claimed, but no information about the particle size of the metoprolol succinate used to prepare the granules is provided.

EP0220143 A discloses controlled release preparation containing a salt of metoprolol **characterized in that** the preparation contains a number of beads comprising a salt of metoprolol as the main soluble component and that said beads are coated with a polymeric membrane containing derivatives of cellulose without protolysable groups and whereby at least 75% of the dose of metoprolol is released within 20 hours virtually independent of the pH in the interval 1-8. The size of the beads in the range of 0.25-2 mm is claimed, but no information about the particle size of the salt of metoprolol used to prepared the beads is provided.

US2005008701 A1 discloses controlled release pellets of metoprolol having an inert core a drug layer optionally comprising a binder and a controlled release coating surrounding that drug layer. The diameter of the inner core is an important feature of the invention. Such pellets are used to prepare extended-release pharmaceutical compositions. Pellet having a diameter of the inert core of less than 30 mesh is claimed, but no information about the particle size of the metoprolol used to prepare the pellets is provided.

EP0311582 A discloses controlled release preparations for administration once daily and containing a combination of metoprolol and a poorly water soluble calcium channel blocking agent of the dihydropyridine type, wherein metoprolol is included in the form of small beads containing as the main soluble component a salt of metoprolol coated with a water-insoluble polymeric membrane and the dihydropyridine is dispersed in a non-ionic solubilizer and whereby both the dispersed dihydropyridine and the beads containing metoprolol are incorporated into a matrix forming a swelling gel in contact with water. No information about the particle size of the metoprolol used to prepare the beads that are incorporated into a matrix forming a swelling gel in contact with water is provided.

WO2004069234 A discloses pharmaceutical compositions comprising a matrix material having metoprolol, or a pharmaceutically acceptable salt thereof, dispersed therein, the dispersion of the metoprolol or pharmaceutically acceptable salt thereof within the matrix material being effective to delay the release profile on administration of the pharmaceutical composition, the tablet being provided with a substantially water-insoluble polymeric coating effective further to delay the release profile on administration of the pharmaceutical composition. No information about the particle size of the metoprolol succinate used to prepare the matrix material is provided.

Time Release Technology also known as sustained-release, extended-release, time-release or timed-release, controlled-release, or continuous-release pills or tablets or capsules formulated to dissolve slowly and release a drug over time. The advantages of sustained-release tablets or capsules are that they can often be taken less frequently than instant-release formulations of the same drug, and that they maintain steadier levels of the drug in the bloodstream.

One of the major problems in the development of extended release pharmaceutical compositions comprising metoprolol succinate is its high solubility, being freely soluble in water. The solubility in water at 37°C is 276 mg/mL (see Ragnarsson et al., International Journal of Pharmaceutics, 1992, vol. 79, n°2-3, pp. 223-232).

For example, the authors of Remington: The Science and Practice of Pharmacy, Lippincott Willians & Wilkins, 20th Edition, page 907, state that "in general, extremes in the aqueous solubility of a drug are undesirable for a formulation into a controlled-release product. A drug with very low solubility and slow dissolution rate will exhibit dissolution-limited absorption and yield an inherently sustained blood level." Also the authors further hold that "for a drug with a very high solubility and rapid dissolution rate, it often is quite difficult to decrease its dissolution rate and slow its absorption. Preparing a slightly soluble form of a drug with normally high solubility is, however, one possible method for preparing extended release dosage forms.

### SUMMARY OF THE INVENTION

The problem to be solved by the present invention is to provide an alternative extended release pharmaceutical composition comprising metoprolol succinate which has a release profile close to zero order, and adequate pharmacokinetic parameters, nevertheless being easy to manufacture and with suitable pharmacotechnical parameters.

The first aspect of the present invention is an extended release pharmaceutical composition comprising metoprolol succinate and at least two pharmaceutically acceptable excipients, wherein one pharmaceutically acceptable excipient is an extended release agent; the second pharmaceutically acceptable excipient is selected from a binder, a diluent and mixtures thereof; and metoprolol succinate is in a crystalline form having a D50 ranging from 5 to 16 microns and a D90 below 50 microns. The inventors have surprisingly found that the behavior of such extended release pharmaceutical compositions comprising metoprolol succinate is just the opposite from that expected by a person skilled in the art. According to common knowledge, the reduction of the particle size, obtained for example using micronization techniques, leads to an increase in surface area and subsequently, according to the Noyes-Whitney equation, to an increase in the dissolution rate (Donald Lee Wise et al., Handbook of Pharmaceutical Controlled Release Technology, CRC Press, 2000, page 345). Therefore these techniques are commonly used when the drug substance has low solubility (Drug Bioavailability, Wiley-VCH, volume 40, page 545). On the other hand, when the drug substance has high solubility, such as metoprolol succinate, the techniques used to formulate it as an extended release pharmaceutical composition are those which reduce the solubility, as for example increasing the particle size or using another less soluble pharmaceutically acceptable salt.

Unexpectedly there are other advantages associated with the present invention. The process is more robust, increasing the reproducibility of the dissolution profile. See for example the reproducibility of the dissolution profile of examples 1 and 2, compared with that of examples 9 and 10. Another improvement was the increase of the metoprolol succinate content of the products ("Assay" according to European Pharmacopeia). This is an important parameter, which according to the Pharmacopeia should be between 90 and 110% for the final dosage form. This means that the assay of the granule must be more restrictive to ensure compliance (normally 95-105%). Using metoprolol succinate having a particle size bigger than that of the present invention the final assay was between 94 and 96%. In contrast the assay of the products of the present invention increases considerably being close to 100%.

The second aspect of the present invention relates to a granule comprising metoprolol succinate and at least two pharmaceutically acceptable excipients, wherein one pharmaceutically acceptable excipient is an extended release agent; the second pharmaceutically acceptable excipient is selected from a binder, a diluent and mixtures thereof; and metoprolol succinate is in a crystalline form having a D50 ranging from 5 to 16 microns and a D90 below 50 microns. Surprisingly, it has been found that the same technical effect is also observed in the granule, before using it to form the extended release tablet.

The third aspect of the present invention is a process for the manufacture of a granule as defined in the previous aspects comprising the steps of:
i) Granulating metoprolol succinate, or a mixture comprising metoprolol succinate and at least one acceptable excipient, by the addition of a binding agent
ii) Optionally sieving or milling the product obtained

The granulation process of the present invention improves the granulation already known in the art, as for example in reducing the granulation time and being easier to calibrate.

The granule obtained can be further mixed with at least one pharmaceutically acceptable excipient and compressed to form a tablet, and preferably the tablet is coated.

### Definitions

As used herein, the term "granulation" refers to the process of agglomerating powder particles into larger agglomerates (i.e. granules) that contain the active pharmaceutical ingredient. The term "granulation" includes wet granulation, dry granulation and melt granulation techniques. The resulting granulated mixture may be further processed, for example, through an extrusion and/or spheronisation process, and/or into various final dosage forms, e.g., capsules, tablets, wafers, gels, lozenges, etc.

The term "wet granulation" refers to any process comprising the steps of addition of a liquid to powder starting materials, preferably kneading, and drying to yield a solid dosage form.

As used herein, the term "melt granulation" refers to the following process that comprises the steps of:
(a) forming a mixture of an active ingredient with at least one granulation excipient;
(b) granulating the mixture at a temperature that is less than or about at the melting point (or melting range) of the active ingredient and
(c) cooling the product,
(d) calibrating of the product obtained after cooling.

The term "dry granulation" refers to any process comprising the compaction or compression of the powder or the starting materials in order to obtain a compacted product, as for example slugs, which further are calibrated to obtain granules with a suitable particle size.

The granulation excipient or excipients,can be present in an amount from about 1% to about 95% by weight of the composition. In one embodiment, the granulation excipient may be present in an amount from about 80% to about 25% by weight of the composition. The active ingredient may be present in an amount from about 5% to about 99% by weight of the composition. In one embodiment, the active ingredient may be present in an amount of about 20% to about 75%.

As used herein, the term "extrusion" is understood as meaning a manufacturing process which is used to process a material or a blend of more than one material through a defined orifice, thereby giving rise to a homogeneous dispersion which possesses an extremely high degree of dispersity (or even a "solid solution"). In particular, in pharmaceutical technology, extrusion comprises an optional blending of different materials if present and the processing of these through a mesh of a sieve or a die of a perforated plate, often including an increase in pressure. The term "melt extrusion" as used herein is understood as meaning a special form of extrusion, where the temperature of the extruded material is raised beyond the melting temperature at a given process pressure of at least one of the components of the processed material. As is well-known to a person skilled in the art, the higher the process pressure, the faster the process is. Consequently, the pressure used in the method of the invention is as high as acceptably possible.

The term "spheronisation" refers to the process of forming spherical particles.

As used herein, the term "granule" is not limited to the agglomerated directly obtained after the granulation processes, but it also refers to the product obtained after the extrusion and spheronisation processes.

The terms "binder" or "binding agent" refer to any substance or mixture that exerts a physicochemical attractive force between molecules, and hence may be used in the formulation of a dosage form. In one embodiment of the invention, the binder or the binding agent may be mixed with other components of the composition, so that it is distributed uniformly throughout the dosage form. In one embodiment the binding agent is formed by the dissolution or dispersion of a binder in a liquid to form a binding solution or dispersion, and preferably the liquid is water. In another embodiment the binder is water. The binder may also provide a matrix upon which any additional components can associate. Binders include, but are not limited to, gelatin, polyvinylpyrrolidone (PVP), hydroxypropyl methylcellulose (HPMC), starch grades (pregelatinized or plain), hydroxypropylcellulose (HPC), and carboxymethylcellulose (CMC).

The term "diluent," as used herein, refers to an agent or mixture of agents that when added to a formulation makes that formulation thinner or less concentrated and may also improve manufacturability. Diluents can be used to stabilize compounds because they can provide a more stable environment. In certain embodiments, diluents increase the bulk of the composition to facilitate compression or create sufficient bulk for a homogenous blend for capsule filling. Such compounds include e.g., lactose, starch, mannitol, sorbitol, dextrose, microcrystalline cellulose such as Avicel ®, dibasic calcium phosphate, dicalcium phosphate dihydrate; tricalcium phosphate, calcium phosphate; anhydrous lactose, spray-dried lactose; pregelatinized starch, compressible sugar, such as Di-Pac ® (Amstar), hydroxypropylmethylcellulose, hydroxypropylmethylcellulose acetate, sucrose-based diluents, confectioner's sugar; monobasic calcium sulfate monohydrate, calcium sulfate dihydrate; calcium lactate trihydrate, dextrates; hydrolyzed cereal solids, amylosem, powdered cellulose, calcium carbonate; glycine, kaolin, sodium chloride; inositol, bentonite, and the like.

The term "extended-release" is to be understood as defined in the United States Pharmacopeia 26, under the General Information section: "extended-release tablets are formulated in such manner as to make the continued medicament available over an extended period of time following ingestion". Extended release is achieved by a special formulation design and/or manufacturing method. The specification for an extended release pharmaceutical composition comprising metoprolol succinate are disclosed in USP 32 (NF27 Vol. 3).

The term "extended release agent" refers to pharmaceutically acceptable excipients, or mixtures thereof, which delay the release of the active substance. These can be hydrophobic materials, and are often hydrocarbons and their derivatives such as lipids, waxes, paraffins and hydrophobic polymers; hydrophilic materials such as cellulose derivatives or methacrylic polymers; and inert non-erodible materials. Examples of "extended release agents" suitable for use herein include poorly water-soluble materials, such as, hydrocolloids, for example, alcohol-soluble cellulose derivatives such as ethyl cellulose and hydroxy-propyl cellulose; water soluble materials such as sodium alginate, pectin, gelatin, carrageenin, arabic acid, agar and karaya; and water insoluble waxes, such as carnauba wax, beeswax and microcrystalline waxes, polyvinyl alcohol, low molecular weight polyethylene, polyvinyl propionate. Preferred are ethylcellulose, sodium alginate and paraffin wax.

As used herein, the term "micronization" refers to a decrease in particle size through application of force to a particle, resulting in the break-up of the particle. Such force may be applied by collision of particles at high speeds.

The dissolution test was carried out according to the European Pharmacopeia 2.9.3. The HPLC detector was set up at λ= 280 nm.

D50 and D90 represent the median or the 50th percentile and the 90th percentile of the particle size distribution, respectively, as measured by volume. That is, D50 (D90) is a value on the distribution such that 50% (90%) of the particles have a volume of this value or less.

The particle size distribution was measured using the following method:
Equipment:
   Beckman Coulter LS13320, module ULM. Measurement range: 0.04 to 2000 microns
   Fluid: Silicone oil
   Optical model: Fraunhofer PIDS included
Preparation of sample:
   - Add 2 to 3 micro-spatulas of sample to a beaker containing 30-40 ml of silicone oil.
   - Re-suspend the sample using a plastic Pasteur pipette, aspirating several times.
   - Stir the sample in a magnetic stirrer for 10 minutes in order to break down the largest lumps.
   - Sonicate for 3 minutes (30KHz, 200W).
   - Place the sample in the analyser
   - Leave the sample to re-circulate in the apparatus for 15 minutes.
   - The sample is now ready for testing in triplicate
Analyser parameters:
   - Pump speed: 60%
   - Optical model: Fraunhofer PIDS included
   - Measuring time: 60 seconds
   - Number of measurements: 3
Determination method:
   - Set the parameters for the analysis of metoprolol succinate:
   - Obscuration required: 7%
   - Graph: frequency by channel and accumulated
   - Interpolation points in percentage: 10, 25, 50, 75 and 95
   - Interpolation points in micron: 1, 10, 50, 100 and 1000
   - Start test
   - Enter sample identification
   - Print out results
NB: Obscuration should be between 4 and 8 %

### DETAILED DESCRIPTION OF THE INVENTION

In one embodiment metoprolol succinate has a D90 below 40 microns. Preferably metoprolol succinate has a D50 ranging from 7 to 12 microns.

Although the desired particle size can be obtained by several methods (e.g. by controlling the crystallization conditions), it has been observed that better results are obtained when the particle size is obtained by micronization.

How to manufacture extended release pharmaceutical compositions is well known. However, excellent results are obtained when the process comprises the step of granulating metoprolol succinate according to the present invention. The extended release agent can be added in several ways. One way would be by coating the granules with an extended release agent. Another would be by the adding the extended release agent during the granulation step. The granulation technique is not limited to wet granulation, but also includes melt and dry granulation. The granule can be used directly after calibrating, to reduce the particle size, or/and can be further processed. An alternative is that the granulation mixture is further extruded and spheronized.

The following examples are set out so as to provide those with ordinary skill in the art with a complete disclosure of how the products claimed herein are prepared, and are intended to be purely exemplary of the invention and are not intended to limit the scope of that which the inventors regard as their invention. Efforts have been made to ensure accuracy with respect to numbers (e.g., amounts, temperature, etc.) but some errors and deviations should be accounted for. Unless indicated otherwise, percentages are by weight, parts are parts by weight, temperature is in °C or is at room temperature, and pressure is at or near atmospheric pressure.

### EXAMPLES

### Preparation of an extended release pharmaceutical composition.

### Example A

| COMPONENT | 190 mg tablet |
|---|---|
| Metoprolol succinate | 190.00 mg |
| Microcrystalline Cellulose PH101 | 591.20 mg |
| Methylcellulose 15 mPa.s | 95.00 mg |
| Glycerol | 1.90 mg |
| Maize starch | 15.50 mg |
| Purified water(*) | - |
| Ethylcellulose 100 mPa.s | 91.40 mg |
| Magnesium stearate | 15.00 mg |
| Isopropanol (*) | - |
| Acetone (*) | - |
| Sub-total | 1000.00 mg |
| Sepifilm® LP 770 White (*) | 30.00 mg |
| Total | 1030.00 mg |

Mainly removed after during steps.

Sepifilm® LP 770 White made up of approx: Hypromellose 6/15 mPa.s (60.00-70.00%), Microcrystalline Cellulose 20 μm (5.00-15.00%), Stearic Acid (8.00-12.00%), Titanium dioxide (E-171) (10.00-20.00%), Purified water (*) 30.00 mg
Method of preparation:
   Batch size: 190 kg of extended release pharmaceutical composition
Machinery used:
   Granulation:
      - Sieve
      - Stainless steel vessel with heating jacket and fitted with stirrer
      - Blender/Kneader
      - Wet granulator with 5 mm mesh screen
      - Fluid bed drier
      - Oscillating sieve
      - Vibrating sieve
Coating of the granulate:
   - Stainless steel vessel
   - Pneumatic propeller stirrer
   - Sieve
   - Pneumatic transfer pump
   - Airtight vessel with slow stirrer
   - Fluid bed equipment
Final blending and compressing of the granulate into cores:
   - Stainless steel sieves
   - Conical blender
   - Rotary tablet press
   - Tablet de-duster
Coating of the cores:
   - Vessel with stirrer
   - 0.1 mm filter
   - Coating equipment

Manufacturing Process:
Granulation:
   1. Check the weights of the starting materials.
   2. Check the cleanliness of the manufacturing area.
   3. Check that all material and equipment are clean and dry.
   4. Sieve metoprolol succinate, microcrystalline cellulose PH101, methylcellulose maize starch and glycerol through a mesh screen.
   5. Load the blender/kneader with the screened starting materials and blend for several minutes with the paddles.
   6. Prepare the binding solution as follows:
      Place purified water into a stainless steel reactor fitted with a heating jacket and stirrer.
      Start the stirring and add the maize starch and glycerol. Stir until complete dispersion.
      Continue stirring and heat to 75-90°C
      Once this temperature is reached and with constant stirring, cool the dispersion to room temperature.
      Once an homogenous, lump-free paste is obtained, dilute the paste to weight with purified water.
      Use immediately after preparation.
   7. Transfer this maize starch paste to the blender/kneader. Knead for several minutes at an impeller speed of 100-300 rpm until a homogenous mixture with a suitable consistency for granulation is obtained.
   8. Screen the mixture through a granulator fitted with a mesh screen.
   9. Load the granulate into the fluid bed drier. Dry at an inlet air temperature of 45-60°C until a residual water content of less than 4.0% is obtained, determined at 100-110°C until constant weight. The approximate drying time is 1-3 hours.
   10. Screen the dry granulate through an oscillating sieve.
   11. Collect the granulate in a duly labelled airtight container.
Coating of the granulate
   1. In a suitable stainless steel vessel containing isopropanol and acetone, add ethylcellulose N-100 and dissolve by pneumatic stirring. Stir until complete dissolution.
   2. By means of a pneumatic pump sieve the solution though a mesh screen and collect in a suitable airtight container fitted with slow pneumatic stirrers (3-8 rpm).
   3. Store the solution in an airtight container until the following day.
   4. To compensate for any loss through evaporation of the solvents, if necessary make up the solution with isopropanol:acetone. Stir for several minutes at 15-30 rpm. Continue stirring at 3-15 rpm during the coating process
   5. Load the granulate in the fluid bed equipment. Coat the granulate until a 26% increase in the theoretical weight is obtained. This percentage has been increased in two examples to evaluate its impact on the dissolution profile.
   6. Coating parameters:
      - Inlet air temperature: 30-50°C
      - Exhaust air temperature: 25-40°C
      - Solution flow rate: 450-550 g/min
      - Inlet air flow rate: 1800-2500 m3/h
   7. Collect the dry coated granulate in a suitable stainless steel container and weigh it.
   8. Sieve microcrystalline cellulose PH101, magnesium stearate through a mesh screen. Sieve the coated granulate through a mesh screen.
   9. Place the coated granulate, and the microcrystalline cellulose PH101 in the blender and blend for 5-15 minutes at 6 rpm.
   10. Add the magnesium stearate. Blend for 4-10 minutes at 5-10 rpm. Weigh the final blend.
Compression
   1. Place the final mixture into the rotary tablet press hopper.
   2. Adjust the parameters. Use oval, concave, 19X10 mm, punches scored on both sides. Compress the granulate into cores.
   3. Determine the yield.
   4. Take samples for Quality Control.
Coating of the cores
   1. Preparation of coating suspension:
      Place purified water into a stainless steel container fitted with a stirrer. Slowly add Sepifilm.
      Stir for at least 45 minutes, until complete dispersion.
      Filter the suspension through a mesh screen.
      Continue stirring during the application of the coating.
   2. Place the cores in the coating pan
   3. Heat the cores to a temperature of 30-45°C, before starting the coating process
   4. Coating process parameters are:
      Pan speed: 5-10 rpm
      Inlet air temperature: 50-65°C
      Exhaust air temperature: 48-62°C
      Spray pressure: 3 bar
   5. Stop the process when the coated tablets have reached a weight increase of approx. 1.5% with respect to the cores.
   6. Dry the coated tablets in the coating pan with intermittent turns until room temperature is reached.
   7. Check the final weight of the coated tablets and calculate the yield.

### Results

The above procedure was repeated with several batches of metoprolol succinate each having different a particle size distribution.

The dissolution profile of the final pharmaceutical composition is given below:

| Particle size distribution API (in microns) | | | | Dissolution value (%) | | |
|---|---|---|---|---|---|---|
| | D10 | D50 | D90 | 8 h | 20 h | Comments |
| Ex. 1 | 1.6 | 7.8 | 34.9 | 49.0 | 78.5 | Micronized API |
| Ex. 2 | 1.6 | 7.8 | 34.9 | 47.9 | 78.4 | Micronized API |
| Ex. 3 | 2.1 | 9.3 | 26.4 | 57.0 | 90.0 | Non-micronized API |
| Ex. 4 | 2.1 | 9.9 | 26.4 | 54.0 | 88.0 | Non-micronized API |
| Ex. 5 | 1.9 | 9.9 | 28.3 | 58.0 | 88.0 | Non-micronized API |
| Ex.6 | 2.6 | 11.0 | 36.2 | 55.2 | 87.4 | Micronized API |

Comparative Examples:

| | D10 | D50 | D90 | 8 h | 20 h | Comments |
|---|---|---|---|---|---|---|
| Ex.7 | 3.0 | 16.2 | 74.8 | 59.0 | 92.0 | |
| Ex.8 | 3.5 | 17.7 | 68.7 | 60.0 | 92.0 | |
| Ex.9 | 3.9 | 17.8 | 73.8 | 63.0 | 94.0 | |
| Ex. 10 | 3.9 | 17.8 | 73.8 | 52.0 | 85.0 | |
| Ex.11 | 3.6 | 18.7 | 67.0 | 67.0 | 98.0 | 26.5% ethylcellulose in the final weight of the granule |
| | | | | | | |
| | | | | | | |
| Ex.12 | 3.7 | 18.8 | 67.1 | 64.0 | 95.0 | 27.0% ethylcellulose in the final weight of the granule |
| | | | | | | |
| | | | | | | |
| Ex. 13 | 5.1 | 20.7 | 42.3 | 62.6 | 87.0 | |
| Ex. 13b | 4.2 | 21.9 | 76.3 | 60.0 | 91.0 | |
| Ex. 14 | 6.0 | 31.0 | 97.8 | 63.0 | 93.0 | |

The assay of the granule is given below:

| | D10 | D50 | D90 | % Assay |
|---|---|---|---|---|
| Ex.15 micronized | 1.9 | 7.9 | 28.7 | 99,8 |
| Ex.16 micronized | 1.9 | 7.9 | 28.7 | 97,0 |
| Ex. 17 non micronized | 2.9 | 19.1 | 84.2 | 95,3 |
| Ex. 18 non-micronized | 2.9 | 19.1 | 84.2 | 94,4 |

## Claims

1. An extended release pharmaceutical composition comprising metoprolol succinate and at least two pharmaceutically acceptable excipients, wherein one pharmaceutically acceptable excipient is an extended release agent; the second pharmaceutically acceptable excipient is selected from a binder, a diluent and mixtures thereof; and metoprolol succinate is in a crystalline form having a D50 ranging from 5 to 16 microns and a D90 below 50 microns.

2. The pharmaceutical composition according to claim 1, wherein metoprolol succinate has a D90 below 40 microns.

3. The pharmaceutical composition according to any one of the claims 1 or 2, wherein metoprolol succinate has a D50 ranging from 7 to 12 microns.

4. The pharmaceutical composition according to any one of the preceding claims, wherein metoprolol succinate has been milled or micronized to reduce the particle size, and preferably it has been micronized.

5. The pharmaceutical composition according to any one of the preceding claims comprising granules comprising metoprolol succinate.

6. A granule comprising metoprolol succinate and at least two pharmaceutically acceptable excipients, wherein one pharmaceutically acceptable excipient is an extended release agent; the second pharmaceutically acceptable excipient is selected from a binder, a diluent and mixtures thereof; and metoprolol succinate is in a crystalline form having a D50 ranging from 5 to 16 microns and a D90 below 50 microns.

7. The granule according to claim 6, wherein metoprolol succinate has a D90 below 40 microns.

8. The granule according to any one of the claims 6 or 7, wherein metoprolol succinate has a D50 ranging from 7 to 12 microns.

9. The granule according to any one of the claims 6 to 8, wherein metoprolol succinate has been milled or micronized to reduce the particle size, and preferably it has been micronized.

10. The use of a granule as defined in any one of the claims 6 to 9, for the manufacture of an extended release pharmaceutical composition.

11. A process for the manufacture of a granule as defined in any one of the claims 6 to 9, comprising the steps of:
i) Granulating metoprolol succinate, or a mixture comprising metoprolol succinate and at least one acceptable excipient, by the addition of a binding agent
ii) Optionally sieving or milling the product obtained

12. The process according to claim 11, further comprising the step of:
iii) Coating the product obtained in the step (ii) with an extended release agent, preferably ethylcellulose

13. The process according to claim 11, wherein the granulation comprises an extended release agent.

14. The process according to any one of the claims 11 to 13, further comprising after step (i) the steps of:
i') extruding the product obtained in the step (i)
i") spheronizing the extruded product obtained in step (i')

15. A process for the manufacture of an extended release pharmaceutical composition comprising the process steps as defined in any one of the claims 11 to 14, wherein the final granule or spheronized granule is further mixed with at least one pharmaceutically acceptable excipient and compressed to form a tablet, and preferably the tablet is coated.

## Patentansprüche

1. Eine pharmazeutische Zusammensetzung mit verzögerter Freisetzung, die Metoprololsuccinat und mindestens zwei pharmazeutisch akzeptable Trägerstoffe umfasst, wobei ein pharmazeutisch akzeptabler Trägerstoff ein Wirkstoff mit einer verzögerten Freisetzung ist; wobei der zweite akzeptable Trägerstoff aus einem Bindemittel, einem Verdünnungsmittel und Mischungen davon ausgewählt ist; und wobei Metoprololsuccinat in einer kristallinen Form vorliegt, mit einem D50-Wert von 5 bis 16 Mikrometer und einem D90-Wert von unter 50 Mikrometer.

2. Die pharmazeutische Zusammensetzung nach Anspruch 1, wobei Metoprololsuccinat einen D90-Wert von unter 40 Mikrometer hat.

3. Die pharmazeutische Zusammensetzung nach einem der Ansprüche 1 oder 2, wobei Metoprololsuccinat einen D50-Wert von 7 bis 12 Mikrometer hat.

4. Die pharmazeutische Zusammensetzung nach einem der vorhergehenden Ansprüche, wobei Metoprololsuccinat zermahlen oder mikronisiert wurde, um die Partikelgröße zu reduzieren, und vorzugsweise wurde es mikronisiert.

5. Die pharmazeutische Zusammensetzung nach einem der vorhergehenden Ansprüche, welche Granulate umfasst, die Metoprololsuccinat umfassen.

6. Ein Granulat mit Metoprololsuccinat und mindestens zwei pharmazeutisch akzeptablen Trägerstoffen, wobei ein pharmazeutisch akzeptabler Trägerstoff ein Wirkstoff mit einer verzögerten Freisetzung ist; wobei der zweite akzeptable Trägerstoff aus einem Bindemittel, einem Verdünnungsmittel und Mischungen davon ausgewählt ist; und wobei Metoprololsuccinat in einer kristallinen Form vorliegt, mit einem D50-Wert von 5 bis 16 Mikrometer und einem D90-Wert von unter 50 Mikrometer.

7. Das Granulat nach Anspruch 6, wobei Metoprololsuccinat einen D90-Wert von unter 40 Mikrometer hat.

8. Das Granulat nach einem der Ansprüche 6 oder 7, wobei Metoprololsuccinat einen D50-Wert von 7 bis 12 Mikrometer hat.

9. Das Granulat nach einem der Ansprüche 6 bis 8, wobei Metoprololsuccinat zermahlen oder mikronisiert wurde, um die Partikelgröße zu reduzieren, und vorzugsweise wurde es mikronisiert.

10. Die Verwendung eines Granulats nach einem der Ansprüche 6 bis 9 für die Herstellung einer pharmazeutischen Zusammensetzung mit verzögerter Freisetzung.

11. Ein Verfahren zur Herstellung eines Granulats nach einem der Ansprüche 6 bis 9, welches die folgenden Schritte umfasst:
i) Granulieren von Metoprololsuccinat oder einer Mischung bestehend aus Metoprololsuccinat und mindestens einem akzeptablen Trägerstoff, durch die Hinzufügung eines Bindemittels.
ii) Optionales Sieben oder Mahlen des erhaltenen Produkts.

12. Das Verfahren nach Anspruch 11, welches darüber hinaus den folgenden Schritt umfasst:
iii) Beschichtung des bei Schritt (ii) erhaltenden Produkts mit einem Wirkstoff mit verzögerter Freisetzung, vorzugsweise Ethylcellulose.

13. Das Verfahren nach Anspruch 11, wobei das Granulieren einen Wirkstoff mit verzögerter Freisetzung umfasst.

14. Das Verfahren nach einem der Ansprüche 11 bis 13, welches nach Schritt (i) die folgenden Schritte umfasst:
i') Extrudieren des bei Schritt (i) erhaltenen Produkts
i") Sphäronisieren des bei Schritt (i') erhaltenden extrudierten Produkts

15. Ein Verfahren zur Herstellung einer pharmazeutischen Zusammensetzung mit verzögerter Freisetzung, welches die Verfahrensschritte umfasst, wie sie in den Ansprüchen 11 bis 14 festgelegt sind, wobei das finale Granulat bzw. das sphäronisierte Granulat noch weiter vermischt wird, mit mindestens einem pharmazeutisch akzeptablen Trägerstoff, und zu einer Tablette gepresst wird, und vorzugsweise ist die Tablette beschichtet.

## Revendications

1. Une composition pharmaceutique à libération prolongée comprenant du succinate de métoprolol et au moins deux excipients de qualité pharmaceutique, dans laquelle un excipient de qualité pharmaceutique est un agent à libération prolongée; le second excipient de qualité pharmaceutique est choisi parmi un liant, un diluant et des mélanges de ces derniers ; et où le succinate de métoprolol se présente sous la forme de cristaux ayant un D50 allant de 5 à 16 microns et un D90 en-dessous de 50 microns.

2. La composition pharmaceutique selon la revendication 1, dans laquelle le succinate de métoprolol a un D90 en dessous de 40 microns.

3. La composition pharmaceutique selon n'importe laquelle des revendications 1 ou 2, dans laquelle le succinate de métoprolol a un D50 allant de 7 à 12 microns.

4. La composition pharmaceutique selon n'importe laquelle des revendications précédentes, dans laquelle le succinate de métoprolol a été moulu ou micronisé pour réduire la taille des particules, et où, de préférence, il a été micronisé.

5. La composition pharmaceutique selon n'importe laquelle des revendications précédentes comprenant des granulés comprenant du succinate de métoprolol.

6. Un granulé comprenant du succinate de métoprolol et au moins deux excipients de qualité pharmaceutique, dans lequel un excipient de qualité pharmaceutique est un agent à libération prolongée ; le second excipient de qualité pharmaceutique est choisi parmi un liant, un diluant et des mélanges de ces derniers ; et où le succinate de métoprolol se présente sous la forme de cristaux ayant un D50 allant de 5 à 16 microns et un D90 en-dessous de 50 microns.

7. Le granulé selon la revendication 6, dans lequel le succinate de métoprolol a un D90 en dessous de 40 microns.

8. Le granulé selon n'importe laquelle des revendications 6 ou 7, dans lequel le succinate de métoprolol a un D50 allant de 7 à 12 microns.

9. Le granulé selon n'importe laquelle des revendications 6 à 8, dans lequel le succinate de métoprolol a été moulu ou micronisé pour réduire la taille des particules, et où, de préférence, il a été micronisé.

10. L'utilisation d'un granulé tel que défini dans n'importe laquelle des revendications 6 à 9, pour la fabrication d'une composition pharmaceutique à libération prolongée.

11. Un procédé pour la fabrication d'un granulé tel que défini dans n'importe laquelle des revendications 6 à 9, comprenant les étapes consistant à :
i) Granuler du succinate de métoprolol, ou un mélange comprenant du succinate de métoprolol et au moins un excipient de qualité pharmaceutique, par l'ajout d'un agent liant.
ii) Optionnellement tamiser ou moudre le produit obtenu.

12. Le procédé selon la revendication 11, comprenant en outre l'étape consistant à :
iii) Enrober le produit obtenu au cours de l'étape (ii) avec un agent à libération prolongée, de préférence de la cellulose éthylique.

13. Le procédé selon la revendication 11, dans lequel la granulation comprend un agent à libération prolongée.

14. Le procédé selon n'importe laquelle des revendications 11 à 13, comprenant en outre après l'étape (i) l'étape consistant à :
i') extruder le produit obtenu au cours de l'étape (i)
i") sphéroniser le produit extrudé obtenu au cours de l'étape (i')

15. Un procédé pour la fabrication d'une composition pharmaceutique à libération prolongée comprenant les étapes du procédé telles que définies dans n'importe laquelle des revendications 11 à 14, dans lequel le granulé final ou granulé sphéronisé est de plus mélangé avec au moins un excipient de qualité pharmaceutique et compressé pour former un comprimé, et le comprimé est de préférence enrobé.
